# EUROPEAN PATENT SPECIFICATION

(11) **EP 0 642 327 B1**
(45) Date of publication and mention of the grant of the patent: **31.07.2002**
(21) Application number: 93913973.9
(22) Date of filing: 18.05.1993
(51) Int. Cl.: A61F 2/28, A61B 17/56

(54) **APPARATUS FOR TREATMENT OF THORACIC DEFORMITIES SUCH AS SCOLIOSIS**
GERÄT ZUR BEHANDLUNG VON THORAXDEFORMIERUNGEN WIE SKOLIOSE
APPAREIL POUR TRAITEMENT DE DEFORMITES THORACIQUES TELLES QUE LA SCOLIOSE

(30) Priority: 19.05.1992 US 885346
(43) Date of publication of application: 15.03.1995
(73) Proprietor: CAMPBELL, Robert M. Jr., San Antonio, TX 78216 (US)
(72) Inventor: CAMPBELL, Robert M. Jr., San Antonio, TX 78216 (US)
(74) Representative: Loven, Keith James
(86) International application number: US9304720
(87) International publication number: WO9322989

(56) References cited:
- US-A- 3 242 922
- US-A- 4 047 523
- US-A- 4 187 841
- US-A- 4 263 904
- US-A- 4 289 123
- US-A- 4 327 715
- US-A- 5 092 889

## Description

This invention relates to apparatus for the treatment of diseases and symptoms evidenced as deformities in the human skeletal systems and particularly for the treatment of scoliosis.

The human spine is a system which consists of a succession of vertebral bodies which, in its normal state, extends within and defines a single sagittal plane. Ideally, there should be substantially no deviation in the frontal plane (perpendicular to the sagittal plane from a straight line. Within the sagittal plane, a certain degree of lumbar lordosis and thoracic kyphosis is normal and desirable. An excess degree of lumbar curvature is known as hyperlordosis, while an abnormally flat lumbar succession of vertebral bodies is known as hypolordosis. In like manner, hyperkyphosis (most commonly seen in Scheuermann's disease) is that condition evidenced by a greater-than-normal degree of curvature in the thoracic spine which gives a hump-back type appearance.

Scoliosis may be defined as lateral deviation and rotation of a series of vertebrae from the midline anatomic position of the normal spine axis. The deformity occurs in three planes - frontal, sagittal and transverse. Scoliosis, in its more severe embodiments, is a debilitating, if not deadly disease. With the progression of the curve, structural changes occur in the vertebrae and in the formation and contour of the rib cage. This, in turn, often threatens respiratory function and capacity. The curvature of the spine itself can pose danger to the spinal cord. Still further, the interrelationships between other thoracic and abdominal organs are changes and the normal function thereof is imperilled. Fully 80% of all scoliosis cases are idiopathic, i.e. the cause is cause unknown.

There is no present "cure" for scoliosis as such, but treatments of the symptoms have been known for some time - treatments with often-times questionable effectiveness, inherent intra-surgical danger to the patient, frequent patient discomfort and/or substantial inconvenience, and substantial likelihood of post-operative complication.

Non-surgical control of scoliosis (as distinguished from correction) is available. Such non-surgical treatments include physical therapy, biofeedback, electrical stimulation and orthosis (the Sayre cast, the Hibbs and Risser casts, the Milwaukee brace, the Boston brace and the Wilmington brace, for example). Reportedly, however, these non-surgical methods can collectively boast, at most, only a 70% success rate in arresting further progression of scoliosis in cases of proven curve progression in growing (relatively immature) spines. Many of these non-surgical methods are contraindicated in cases involving curvatures greater than a specific range (usually about 40 degrees), certain patients with physical infirmities in addition to the scoliosis, patients with certain remaining growth potential, and/or with patients who cannot be reasonably expected to rigorously follow a prescribed therapeutic regimen or to emotionally tolerate the limitations and appearances of the various braces.

Surgical intervention in the correction of scoliotic curvature presently involves spinal instrumentation and spinal fusion first pioneered by Hibbs, et al (see Hibbs, RA, Risser, JC and Ferguson, AB: "Scoliosis treated by the fusion operation." J. Bone Joint Surg., 6:3, 1924). One without the other is generally viewed as ineffective under current convention. The over-all objective of the surgical intervention is to correct the scoliosis as much as is possible, and to restore compensation of the spine with a symmetrical trunk and with head, neck and shoulders centered over the pelvis; and to stabilize the spine and prevent curve progression. The objective of the spinal instrumentation portion of surgical treatment of scoliosis is to immediately correct curvature to the degree possible and to immobilize the spine in the corrected orientation until a solid fusion has taken place.

Problems abound with currently available spinal instrumentation. Some instrumentation occupies space needed for the bone graft placed over the posterior spine. Also, the attachment means used for spinal instrumentation inherently risks intraoperative spinal cord damage with the potential for irreversible paralysis. Still further, many spinal instrumentation systems are prone to disengagement because their attachment schemes involve screws (prone to disengage from atrophic vertebrae) or hooks which only partially encircle vertebral body projections (prone to dislodging during movement).

"Harrington Instrumentation" (see Harrington, P.R.: Treatment of Scoliosis. Correction and internal fixation by spine instrumentation. J. Bone Joint Surg., 44-A:591, 1962, and also Harrington, P.R.: Surgical instrumentation for management of scoliosis. J. Bone Joint Surg., 42-A:1448, 196.) is the posterior spinal instrumentation by which all current systems are compared. In this system, bone-purchasing hooks are attached to posterior elements of the spine facets, laminae, and transverse processes. Through these hooks, distraction forces are applied to the concave side of the spinal curve by the ratchet principle, and compression forces are applied on the convex side of the thoracic curve at the base of the transverse processes and adjusted by the threadnut principle.

Despite its prominence, the Harrington system has drawbacks: (1) failure of derotation of the spine as the distraction force straightens the lateral curvature; as a result the rib hump is not corrected; (2) the distraction forces of the Harrington instrumentation flatten the spine with the result that the normal lumbar lordosis is obliterated thereby producing a marked deformity; (3) Harrington instrumentation does not provide enough stability to the spine and, therefore, postoperative immobilization is required in the form of a cast or spinal orthosis; (4) the bulky nature of the Harrington instrumentation is such that it protrudes well beyond the normal dorsal contour thereby "tenting up" the overlying skin and causing breakdown problems; and (5) Harrington instrumentation does not accommodate growth in cases of implantation in children; and (6) direct exposure of the spinal components to any spinal instrumentation often promotes inadvertent spinal fusion.

A relatively new system (the Cotrel-Dubousset (C-D) Instrumentation - see Cotrel, Y. and Dubousset, J.: New Segmental posterior instrumentation of the spine. Orthop. Trans., 9:118, 1985) is a segmental spinal instrumentation that addresses many of the Harrington system's shortfalls. Nevertheless, C-D instrumentation exhibits its own shortcomings, the principle one being that it is complex-and cumbersome, with too many "moving parts". Also, implantation is extremely complex and requires the skills and experience possessed by few practitioners. It obscures the posterior spine; limiting the amount of bone graft surface for biologic fusion.

One current form of anterior segmental instrumentation for treatment of scoliosis is the Zielke instrumentation (see Zielke, K. and Pellin, B.: Neue Instrumente and Implantate zur Erganzung des Harrintson Systems. Z Orthop. Chirl, 114: 534, 1976). This system, however, obviously involves accessing the anterior surfaces of the spinal column to attach instrumentation. This, in turn, carries a significant risk of neural and vascular injury with the accompanying risk of partial or total paralysis.

The history of scoliosis and its treatment yields as its less on a seeming paradox: it is dangerous (and often ineffective) to treat the spine in addressing spine anomalies. This paradox has heretofore remained unsolved for two primary reasons: (1) the causation and mechanisms of scoliosis are not understood and, therefore, cannot be addressed in a preventative or even curative manner; and (2) direct management of an affected system (in this case the spine) is the traditional approach common to virtually all orthopedic procedures, a predisposition which yields a myopic view of the possible remedies as evidenced by the spinal instrumentation of the prior art.

The Applicant has already taught, in his U.S. Patent No 5, 092, 889, the design of a prosthetic rib which is adjustable in length and comprises three principal components, that is a rib sleeve carriage attachment, a rib shaft/rib shaft carriage attachment and a rib sleeve. The effective length of the prosthetic rib is determined by the degree to which the rib shaft is telescopically received within the rib sleeve, a lock being used to secure the rib shaft and the rib sleeve together after telescopic adjustment to provide a desired length for the prosthetic rib. The rib shaft and rib sleeve are formed whereby they jointly define a single arc having a constant radius of curvature regardless of the degree the rib shaft is received within the rib sleeve. This radius of curvature may be adjusted in the manufacturing process according to the expressed preference of the responsible surgeon, as dictated by the physiology of the intended recipient. The rib sleeve carriage attachment and the rib shaft carriage attachment are each formed as a pair of rods which are manipulated by the surgeon to encircle the appropriate human rib.

This prosthetic rib is implanted, in a position spaced laterally from the spine, to span between existing natural ribs thereby compensating for an abnormal absence of intervening ribs.

Applicant's U.S. Patent No. 5, 092, 889 therefore teaches a prosthetic rib comprising a first element having a first attachment means for attachment to a first human rib and a second element telescopically received within a portion of the first element and having a second attachment means for attachment to a second human rib.

The present invention is concerned with the provision of apparatus for the treatment and management of thoracic deformities such as scoliosis through manipulation, not of the spine, but of the adjacent ribs. Such apparatus is hereinafter referred to as a "thoracodorsal distractor" and comprises a telescopically expandable strut which is attachable at a first of its two ends to an upper thoracic human rib immediately adjacent to the spine and at the second end to a lower human rib adjacent to the spine.

According to the present invention, an apparatus for attachment between human ribs is a thoracodorsal distractor having a first upper length of the distractor contoured to define a first radius of curvature, and a second lower length of the distractor contoured to define a second radius of curvature which is larger than the first radius of curvature. In this manner the thoracodorsal expander is contoured for initially conforming to a merely slightly corrected scoliotic deformity and thereafter, through successive adjustments, for defining and imposing upon the patient's dorsum a substantially normal dorsal contour from the thoracic to the sacral segments thereof. The thoracodorsal distractor thereby defines a contour in the sagittal plane which exhibits a complex curve. Consistent with the curvature defined by the trough or "posterior gutter" which is collectively defined by the ribs' arcuate deviation lateral to the spinal column and medial to posterior angle of each rib, the upper portion of the thoracodorsal distractor which is to overlie the upper thoracic ribs defines a curvature in the sagittal plane of a smaller radius than that of the remaining extent (the lower portion) of the distractor which is to overlie the more lower thoracic and the lumbar regions of the dorsum. The upper portion of the distractor is of a fixed length and contour, while the lower portion is telescopically adjustable to expand and contract within a single arc of curvature of substantially larger radius than that of the upper portion.

Each attachment means may comprise at least one rod affixed at the outer end of the respective element, each rod being made of malleable material and being of a length whereby it may be manipulated to loosely encircle a human rib. Alternatively each attachment means may comprise a pair of rods affixed at the outer end of the respective element, each pair of rods being made of a malleable material and being of a length whereby they may be manipulated to loosely encircle a human rib. The first attachment means may comprise first and second members which are relatively movable between a first position in which they define a closed circle of a size for loosely encircling the human rib and a second position in which they define a broken circle having a breach of sufficient size to admit the upper human rib.

The distractor may further comprise locking means for selectively arresting telescopic movement between the first element and the second element at each of a plurality of post-operatively selectable positions. A rib sling may be provided for attaching a medial portion of the apparatus to a third human rib underlying the medial portion and for forcibly drawing a portion of the third rib to a position adjacent to the apparatus. A rack and pinion system may be operatively arranged to react between the first and second elements to adjust the spacing between the first and second attachment means. The pinion is preferably removable after the spacing has been adjusted.

A locking means is preferably arranged to lock the first and second elements together after the spacing between the first and second adjustment means has been adjusted. This locking means may either be a ratchet mechanism which inhibits contraction of the distractor whilst allowing its extension, or may be a locking disc arranged to lock the first element to the second element at each of a plurality of post-operatively selectable positions.

One mode of using the thoracodorsal distractor involves placement of two distractors, one on either side of the scoliotic spine. One distractor is to act as a distractor (on the concave side of the curvature), the other as a means of compression (on the convex side). In the former instance, the distractor may be implanted in a contracted or shortened state. The distractor will be sized to exert a distractive force on the sacrum and the upper attachment rib thereby tending to straighten the intervening length of the spinal column. The distractor can subsequently be expanded in length through successive post-implantation adjustments as the scoliotic curvature is gradually corrected.

On the convex side of the scoliotic curvature, the opposite is true - the distractor may be implanted in a relatively expanded state to exert a suitable initial compressive force between the upper attachment rib and the lower rib. This directly compliments and supplements the corrective action of the distractor on the concave side of the curvature. As the dynamic treatment progresses, the convex side distractor can be shortened through successive adjustments.

The distractors can further serve as a platform from which metallic slings are suspended to encircle and draw toward the distractor selected, mis-oriented ribs which lie thereunder. In this manner, the distractors serve to derotate vertebrae to which the ribs manipulated in this manner are attached.

Applicant's thoracodorsal distractors present drastic improvements over the scoliotic treatment instrumentation and methods of the prior art. By attaching to the ribs adjacent the spine, rather than to the spine itself, substantial risk of irreversible neural injury is eliminated. The positioning of the implanted distractors allows ready access to the spine for spinal fusion procedures. In patients, because of skeletal immaturity, use of the distractor reduces the chance of inadvertent spinal fusion as a result of direct exposure of the spine to the instrumentation. Furthermore, the expandability of the thoracodorsal distractors allows dynamic treatment of scoliosis as well as the accommodation of growth when implanted in children. Further still, the size and contour of the distractors also reduce the likelihood of skin necrosis which attends use of the bulky, protruding instrumentation of the prior art.

Such benefits are inherent in the thoracodorsal distractors regardless of the attachment means (so long as this is stable as to the upper rib and lower rib). The distractor can also extend from a lower rib to the sacrum for the purpose of treating lumbar scoliosis. Nevertheless, Applicant has derived rib and sacral attachment means which are both virtually infallible (subject to bone integrity) and relatively easy to operate. These improvements, which are described in detail in the detailed description, represent a departure from and an improvement over Applicant's prior inventions, the embodiments of which attach to ribs and require considerably advanced skill and experience to implant safely and effectively.

Applicant also provides a length adjustment system for his thoracodorsal distractors which is, after implantation, very simply operable through very small puncture wound-like incisions.

The invention is now described, by way of example only, with reference to the accompanying drawings, in which : -
Fig. 1 is an elevational side view of a thoracodorsal distractor of Applicant's invention (with simple rod-type rib carriages) in a contracted, or shortened configuration.
Fig. 2 is the thoracodorsal distractor of Fig. 1 shown in top plan view.
Fig. 3 is a side elevational view of the distractor of Figs. 1 and 2 shown in a telescopically expanded configuration.
Fig. 4 is a cross sectional view of the distractor sleeve at line 4 - 4 of Fig. 3.
Fig. 5 is a cross sectional view of the distractor shaft along line 5 - 5 of Fig. 3.
Fig. 6 is a perspective anatomical view of one thoracodorsal expander implanted on one side of a patient's spinal column.
Fig. 7 is an elevational, partially sagittally cross sectional view of the upper portion of a thoracodorsal distractor of Applicant's invention. This view depicts the increased curvature of the upper portion of the distractor so as to accommodate the more curved contour of the more upper thoracic ribs at the distractor's line of attachment thereto.
Fig. 8 is a side elevational view of a segment of a thoracodorsal distractor as connected to a rib (shown in cross section) by way of a metallic rib sling.
Fig. 9 is a side elevational view of the auto-lock rib carriage of Applicant's preferred embodiment
Fig. 10 is the auto-lock rib carriage of Fig. 9 with0 the upper carriage in the closed position.
Fig. 11 is an elevational front view of the auto-lock rib carriage of Fig. 9 with the over carriage in the open position.
Fig. 12 is a elevational front view of the auto-lock rib carriage of Fig. 9 with the over carriage in the closed position.
Fig. 13A is a front elevational view of the saddle lock for use with the auto-lock rib carriage of Fig. 9.
Fig. 13B is a rear elevational view of the saddle lock of Fig. 13A.
Fig. 13C is a side elevational view of the saddle lock of Fig. 13A.
Fig. 14A is a top plan view of the carriage shaft lock for use with the auto-lock rib carriage of Fig. 9.
Fig. 14B is a front elevational view of the carriage shaft lock of Fig. 14A.
Fig. 15 is a bottom plan view of a portion of a thoracodorsal distractor having Applicant's improved expansion system.
Fig. 16 is a top plan, partial cut-away view of the portion of the thoracodorsal distractor of Fig. 15.
Fig. 17 is an enlarged portion of Fig. 15 with the distractor shaft removed for visibility of the rack portion of Applicant's improved expansion system.
Fig. 18 is a cross-sectional view (perpendicular to the long axis of the distractor) of a portion of Applicant's improved expansion system with the geared probe shown close to operative position for operation of the system.
Fig. 19 is a side elevational view of the probe used in Applicant's improved expansion system for the thoracodorsal distractor.
Fig. 20 is an end elevational view of the tip .of the probe of Fig. 19 showing the gear and pin affixed at the lower end thereof.
Fig. 21 is a cross-sectional view (perpendicular to the long axis of the distractor) of a portion of Applicant's improved expansion system.
Fig. 22 is a side elevational view of a portion of Applicant's improved expansion system showing operation and position of the ratchet mechanism portion of such system.
Fig. 23 is a bottom plan view of the ratchet mechanism component of Applicant's improved expansion system for his thoracodorsal distractor.
Fig. 24 is an end elevational view of the ratchet mechanism of Fig. 23.
Fig. 25 is an elevational, partially cross-sectional view of the micro access distraction lock component of Applicant's improved expansion system for his thoracodorsal distractor.
Fig. 26 is an exploded, side elevational view of the locking disk and hex screwdriver components of the micro access distraction lock component of Applicant's improved expansion system for his thoracodorsal distractor.
Fig. 27 is a bottom plan view of the threaded cap disk of the locking disk of Fig. 26.
Fig. 28 is a bottom plan view of the threaded cap disk of the locking disk of Fig. 26.

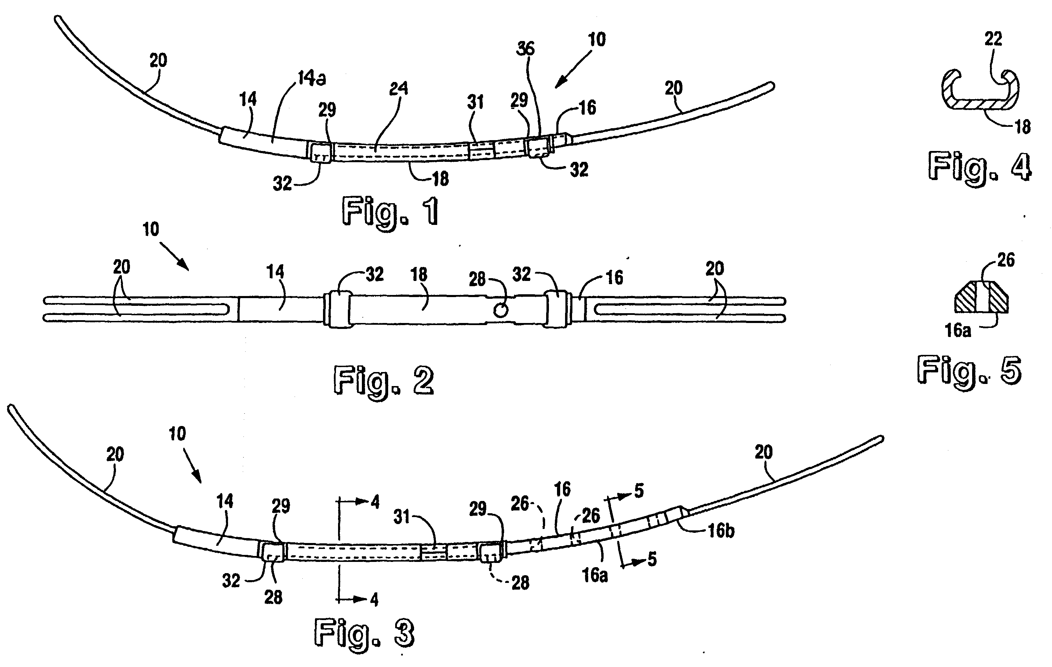

Referring to FIG. 1, a first embodiment of the device of Applicant's invention (hereinafter usually referred to as the "thoracodorsal distractor") is identified generally by the reference numeral (10). This embodiment is the simplest one and is designed, by virtue of its attachment means (to be identified later) to be attached to upper thoracic and lower lumbar natural ribs. Alternative embodiments with different attachment means at the lower end of the distractor will be discussed later herein.

The thoracodorsal distractor (10) is designed to be adjusted in length subsequent to implantation. The primary purpose of the adjustability being to accommodate growth of a child in whom the thoracodorsal distractor (10) is implanted or to allow for dynamic treatment scoliosis and other spinal anomalies (to be discussed later). The adjustability is also a benefit in using a single sized thoracodorsal distractor (10) for applications requiring varying distractor lengths. This permits use of a single sized distractor (10) in a single patient in different positions or in different patients with varying physiological dimensions. Both of these scenarios have obvious financial benefits to the patient(s) when compared with having a number of custom fabricated prostheses made for very specific, limited applications.

Referring to FIGS. 1, 2, and 3, the thoracodorsal distractor (10) comprises three principal components: a upper distractor sleeve carriage attachment (14), a distractor shaft/lower distractor shaft carriage attachment (16), and a distractor sleeve (18). The distractor shaft/lower distractor shaft carriage attachment (16) is a single object of unitary construction, but for discussion purposes may be divided between the distractor shaft (16a) and the lower distractor shaft carriage attachment (16b).

Unless otherwise specified, all components of the thoracodorsal distractor (10), except the distractor sleeve (18) which is made of Titanium Alloy 64, are manufactured of Commercially Pure (CP) Titanium. The use of titanium is dictated by the strength and flexibility requirements for the components of the thoracodorsal distractor (10) in light of the dimensions of such components. Other materials, such as surgical grade stainless steel, may be used in constructing the thoracodorsal distractor (10), but at the expense of the optimum balance of benefits derived from titanium. Another benefit arising from the use of titanium is derived from the fact that it is not a ferromagnetic metal. As such, titanium is compatible with magnetic resonance imaging (M.R.I.) scanning, a much preferable diagnostic procedure, particularly with patients who would normally be considered as recipients of Applicant's thoracodorsal distractor (10).

The upper carriage attachment (14) is provided with a pair of rods (20) and the lower carriage attachment (16b) is provided with a similar pair of rods (20). The rods (20) serve as the attachment means for anchoring the thoracodorsal distractor (10) to natural ribs (12) and will be discussed in detail hereinafter.

Referring again to FIGS. 1, 2, 3 and 4, the distractor sleeve (18) may be described as an elongate, semi-oval partial conduit with a lengthwise oriented channel (22) interrupting the lower surface of the distractor sleeve (18). The presence of the channel (22) is in response to manufacturing cost limitations. It should be understood that a suitable alternative sleeve which lacks the channel (22) entirely (not shown in the drawings) would be acceptable for the purposes stated herein, but would be available, if at all, at a considerably higher price because of difficulties in manufacturing such a sleeve. For that reason, the depicted distractor sleeve (18) would be considered a preferred embodiment.

Referring to FIGS. 1, 2, 3 and 5, the distractor shaft (16a) is of solid construction and has a lengthwise oriented ridge (not visible in the drawings). The ridge is designed to mechanically interface with the channel (22) when the distractor shaft (16a) is telescopically received within interior lumen (24) of the distractor sleeve (18) as it is designed to do. While the presence of the channel does tend to weaken the distractor sleeve (18) in resisting axial rotation relative to the distractor shaft (16a), or vice versa, when a torque is applied to either, the restraining action of the ridge's interface with the channel (22) compensates completely for any such tendency. Prior to incorporating the ridge into the distractor shaft (16a) design, experimentation revealed a marked tendency toward such relative rotation particularly when the thoracodorsal distractor (10) was extended to near its maximum extent.

The distractor shaft (16a) and the distractor sleeve (18) are formed whereby they jointly define a single arc having a constant radius of curvature in a single plane regardless of the degree the distractor shaft (16a) is received within the distractor sleeve (18). The radius of curvature of the distractor sleeve (18) and distractor shaft (16a) may both be adjusted in the manufacturing process according to the expressed preference of the responsible surgeon, as dictated by the physiology of the intended recipient

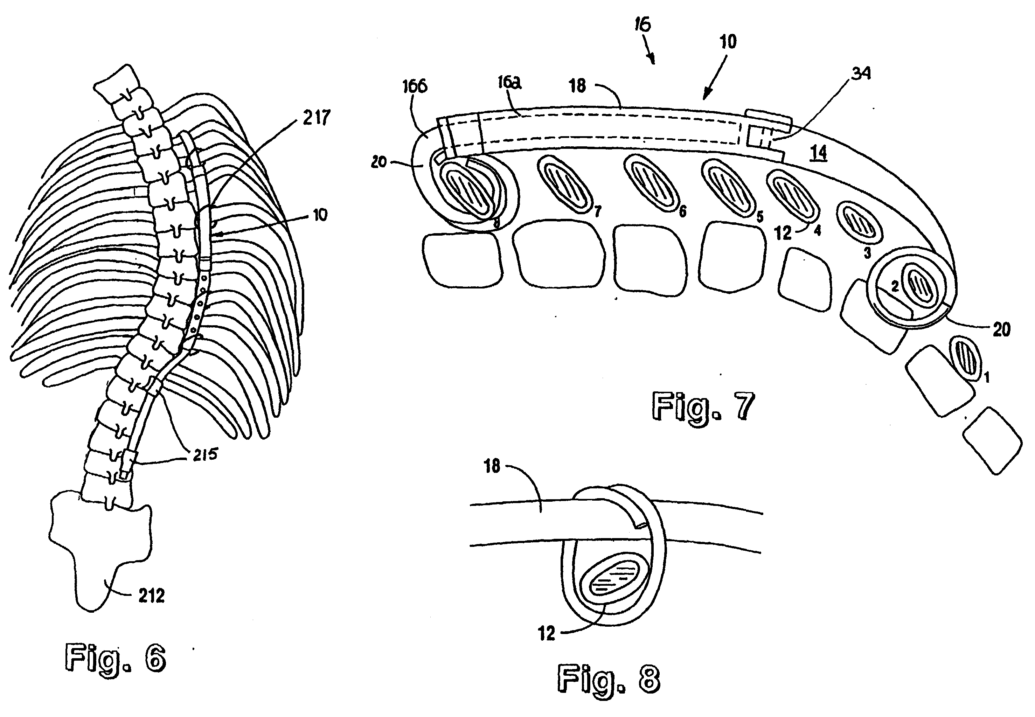

Referring principally to Figures 1 and 7, the upper distractor sleeve carriage attachment (14) projects through the same plane as the distractor sleeve (18) and distractor shaft/lower distractor shaft carriage attachment (16) and is also arcuate in configuration. However, the upper sleeve carriage attachment (14) has a curvature of smaller radius than that of the distractor sleeve (18) and distractor shaft/lower distractor shaft carriage attachment (16) so as to conform to the contour defined by portions of the more upper, thoracic natural ribs (12) over which the thoracodorsal distractor (10) extends once implanted (as in Figure 7). Both the upper and lower portions of the thoracodorsal distractor (10) are intended, in practicing the Applicant's methods for treating and managing scoliosis, to extend along the trough or "posterior gutter" collectively defined by the ribs on one side of and immediately adjacent to the spine as illustrated in FIG. 6. The lower portion of the distractor (10) mimicks a conventional segmental spinal hook system attachable directly to the spine by hooks (215) (such as a Harrington rod system). Metallic rib slings (217) are shown connecting intermediate ribs to the distractors.

By way of example only, Applicant has determined for one patient, a juvenile (at the time of this Application, not yet having received a thoracodorsal distractor) that the appropriate radius of curvature for the upper distractor sleeve carriage attachment (14) is approximately 13.5 cm while that of the distractor shaft/lower distractor shaft carriage attachment (16) is approximately 40 cm.

Referring principally to FIGS. 1 and 3, the effective length of the thoracodorsal distractor (10) is determined by the length of the distractor sleeve (18) and the degree to which the distractor shaft (16a) is telescopically received within the distractor sleeve (18). The fixed length of the upper distractor sleeve carriage attachment (14) is, of course, also partially determinative.

The variable spatial separation of the natural ribs (12) to which the implanted thoracodorsal distractor is attached is but one advantage of Applicant's design. It is important to note that as the distractor 10 is lengthened (as in the case where the distractor (10) is implanted on the concave side of a scoliotic curve wherein it is used to distract natural ribs (12) on either side of the curve and to thereby force the spinal column into a more straight orientation), its medial portions move posteriorly relative to the patient's central anatomical axis. This is a function of the arc in which the lower portions of the distractor, as formed by the distractor sleeve (18) and the distractor shaft (16a), extend as they telescopically expand. Consequently, when natural ribs are tethered to the distractor (10) (as in Figure 6), they are drawn outward so as to more properly align them and to derotate the vertebrae to which these ribs (12) are attached. The combination of the distraction of the marginal attachment ribs (12) and the derotation of the spine as just described provides a highly effective treatment modality for the deviations in all planes associated with scoliosis.

To secure the relative positions of the distractor shaft (16a) and the distractor sleeve (18) once a desired length is attained, the distractor shaft (16a) has a plurality of evenly spaced holes (26) passing there-through. The distractor sleeve (18) of one embodiment has two holes (28) spaced complementarily to the holes (26) in the distractor shaft (16a). The holes (28) in the distractor sleeve (18) are situated on the outer face of the distractor sleeve (18). The upper distractor sleeve carriage attachment (14) also has one hole (30) passing through its sleeve engaging projection (14a).

The holes (26), (28), and (30) are oriented whereby a linear object may concurrently extend through one of the two holes (28) in the distractor sleeve (18) and one of holes (26) in the distractor shaft (16a) when the distractor shaft (16a) is telescopically received within one end of the distractor sleeve (18). Likewise, a second linear object may extend through the other hole (28) in the distractor sleeve (18) and through hole (30) in the upper distractor sleeve carriage attachment (14) when the sleeve projection (14a) is telescopically received and properly positioned within the ether end of the distractor sleeve (18).

Referring principally to FIGS. 1 and 3, once the distractor shaft (16a) and the distractor sleeve (18) are properly, relatively positioned, they are secured using a distraction lock (32). One embodiment of the distraction lock (32) includes a pin (not separately identified in the drawings) long enough to extend through either holes (28) and (26) or through holes (28) and (30) when in position on the assembled thoracodorsal distractor (10), but not long enough to extend beyond the termini of the gripper flanges of the locks (32). The tip of the distraction lock's (32) pin as well as the terinini of its gripper flanges are rounded. The limit on the length of the pin (34) and the just-referenced rounding are in satisfaction of safety concerns. Sharp edges and slender protrusions are to be avoided in anticipation of the distraction lock (32) possibly becoming dislodged after implantation and have been so avoided in Applicant's preferred embodiment of the distraction lock.

Referring principally to FIGS. 1 and 3 the distractor sleeve (18) has two pairs of recesses (29) with which the distraction locks (32) are designed to mate. Each recess (29) is formed having a first zone with a depth such that the gripper flanges (36) of a distraction lock (32) must yield slightly to pass thereover, this zone being nearer the top of the distractor sleeve (18). A second zone (31), slightly deeper into the distractor sleeve (18), is separated by a palpable line of demarcation visible in FIGS. 1 and 3 and lies closer to the bottom of the distractor sleeve (18). The gripper flanges (36) "snap" into the lower, deeper portion of their respective recesses (29) when a distraction lock (32) is installed. In this manner, the distraction lock (32) is securely held in place until or unless pried from the distractor sleeve (18). An alternative embodiment of the distractor sleeve (18) (not shown in the drawings) incorporates multiple pairs of recesses (29) and associated holes (28) near one end of the distractor sleeve (18). Such a distractor sleeve (18) may be shortened using a hack saw at the time of surgery to shorten the starting, most retracted over all length for the thoracodorsal distractor (10) leaving a fully functional distractor sleeve end having the neces sary pair of recesses (29) and hole (28). When shortening this embodiment of the distractor sleeve (18), the distractor sleeve (18) is simply cut at a point between adjacent pairs of recesses (29) and the cut end is then smoothed using a file. Such an alternative embodiment of the distractor sleeve (18) permits its use in situations which otherwise would require the manufacture of a shorter distractor sleeve (18). Wider applicability for any one component of Applicant's invention has obvious financial benefits to recipient patients.

The holes (26) in distractor shaft (16a) in the preferred embodiment for use in very young children have been spaced in 10mm intervals in anticipation of the likely growth intervals which will indicate an adjustment of the thoracodorsal distractor (10). Such spacing is in recognition of the fact that only slight misalignment of the spinal column can result in discomfort and possible spinal cord injury.

Referring principally to FIGS. 2 and 7, both the upper distractor sleeve carriage attachment (14) and the distractor shaft carriage attachment (16b) (of the simplest embodiment of the thoracodorsal distractor (10)) include two rods (20) at their respective ends. The rods (20) are round in cross section. The rods (20) have a cross sectional diameter of 2mm in the preferred embodiment. The rods' (20) round cross sectional shape was chosen as a means for minimizing the biological trauma to the periosteum of the ribs (12) and to the inferior surfaces of the ribs (12) where the rods (20) have their primary contact therewith (to be discussed in more detail hereinafter).

The specific 2mm diameter of the rods (20) was chosen after numerous alternative specifications were tested. A 2mm diameter of CP Titanium has proven to provide the optimum balance between the flexibility necessary for safe manipulation during implantation and strength necessary for post-implantation stability. No other material tested in a 2mm rod configuration and no other dimension in CP Titanium provided the preferred characteristics for the rods (20).

The rods (20) of the preferred embodiment are 76mm in length. This length has been shown through experimentation to provide a quite acceptable degree of surplus length to facilitate the needed manipulation during implantation both to circumvent the natural ribs (12) at the basic level, as well as to adjust the orientation and position of the loops formed from the rods (20) in determining the over-all orientation of the thoracodorsal distractor (10) within the patient. The indicated length does not, however, introduce excessive length which would impede manoeuvring during implantation and require excessive bending to avoid surrounding tissues.

Referring principally to FIG. 7, the rods (20) are during the implantation procedure manipulated by the surgeon to circumvent the appropriate natural rib (12). The path of the rods (20) about the natural rib (12) is essentially circular when properly implanted, even though the rib would be' better described as oblong. This is an important aspect of practicing Applicant's invention for several independently significant reasons. The circular circumvention permits the carriage attachments (14) and (16b) to pivot relative to the natural ribs (12). This is, important, in part, because the carriage attachments (14) and (16b) change orientation relative to the ribs (12) to which they are attached as the length of the thoracodorsal distractor (10) is changed subsequent to implantation.

The ability of the carriage attachments (14) and (16b) to pivot is further important in allowing the thoracodorsal distractor (10) to partially accommodate traumatic force which may occur in falls, etc. while not transferring the force to the natural ribs (12) in a manner which would likely fracture the natural ribs (12) or damage the spine to which the distractor (10) is so closely attached. If the carriage attachments (14) and (16b) were rigidly attached to natural ribs (12), the carriage attachments (14) and (16b) would apply a possibly damaging torque to the natural ribs (12) in response to a traumatic force to the distractor shaft (16a) and/or distractor sleeve (18). This is substantially avoided by the circular path of circumvention suggested herein. Also, the relatively loose circumvention of the natural ribs (12) tends to "cage" the rib, not clamp it, obviating the danger of rib ischemia at the site of contact between the rods (20) and the natural rib (12) surface. Still further, the gentle movement permitted by the preferred mode of attachment for the thoracodorsal distractor (10) and brought about by normal movement of the recipient has the tendency to promote work hypertrophy thereby actually strengthening the natural rib (12).

When the thoracodorsal distractor (10) is properly implanted and adjusted, the principal contact of the rods (20) with the natural ribs (12) is to inner surface areas of the natural ribs (12) relative to the intervening chest wall defect. In this manner, the rods (20) "cradle" the natural ribs (12) at a point of minimum contact as opposed to deleteriously compressing them.

The rods (20) number two for each of the carriage attachments (14) and (16b) in satisfaction of some of Applicant's material objectives in designing the preferred embodiment. Most notably, dual attachment sites for the carriage attachments (14) and (16b), as opposed to a singular attachment site, provide substantial rotational stability for the thoracodorsal distractor (10). By way of comparison, a single site of attachment will do little to stabilize the thoracodorsal distractor (10) against even minor deflective forces while a dual attachment quite ably resists such force. Also, the cumulative mass of titanium needed for strength of the attachment to the natural ribs (12) can be divided between the two rods (20) as opposed to being embodied in a single, larger rod. Such a single rod would be too stiff to safely manipulate during implantation if it incorporated the same quantum of titanium as is divided between the two rods (20) of each carriage attachment (14) and (16b) of the preferred embodiment.

The use of three or more rods (20) is not recommended because of the associated consumption of surface space on the natural ribs (12) and the minimal additional stability which would be achieved. Because a plurality of thoracodorsal distractors (10) will be required in most situations requiring any use of the thoracodorsal distractor (10), conservation of natural rib (12) surface space is desired.

A sample surgical procedure involved in implantation of the thoracodorsal distractor is outlined as follows:
Three 4 cm longitudinal skin incisions are required. The first incision is made at the base of the thoracic spine. It is carried down to the paraspinal muscles overlying the posterior spinous processes of the thoracic spine. The soft tissues and the osseous elements of the spine are not violated in order to minimize the risk of accidental spine fusion. Dissection then continues laterally over the top of the paraspinous muscles to avoid denervation of the muscle. This carries the incision deep to the medial rib at its intersection with the transverse process.

The selected point of rib attachment is chosen and a periosteum incision made over the rib. The retractors are inserted to elevate the periosteum off the rib and this protects both the inferior neurovascular bundle of the rib and also the underlying lung. Pneumothorax is the only serious complication of the operative procedure and the risk is minimal with subperiosteal direction which orthopedists are very familiar with. Once the rib site is prepared, then a second and third site is prepared both at the central portion of the thoracic spine and at the upper portion of the spine. The surgical sites involve reflection of the trapezius muscles laterally from the central and inferior operative sites, and in addition, the levator scapuli and rhomboid muscles are reflected laterally at the superior prosthesis attachment site. Next, a malleable rod of similar dimensions of the prosthesis is threaded carefully from the central operative exposure site lowerly, until visible in the lower site. A similar rod is then threaded through the central site upperly. A thick plastic tube is then anchored to the first malleable rod at the lower site and then threaded up to the central site. Next, the plastic tube is then anchored to the upper malleable rod and threaded out to the upper operative site. Next, the prosthetic sleeve and shaft are attached to the plastic at the inferior site and manipulated through both twisting and gentle pushing up the paraspinal gutter until it is in the proper position for implantation. The plastic tube is pulled during this manoeuvring and it guides the prosthesis through the hole made by the malleable rods and prevents the upper end of the prosthesis from accidental plunging into the thorax and causing either cardiac or pulmonary damage (one known complication of subcutaneous threading of a straight Harrington or Moe rod in this fashion is plunging of the sharp straight end of the rod into the chest as it attempts to pass over the apex of the thorax of the central thoracic spine). Once in position, the rib carriages are engaged to the upper and lower ribs exposed by prior subperiosteal direction. If a titanium sling loop is necessary at the central incision, then it is threaded around a prepared rib and loosely around the prosthetic sleeve.

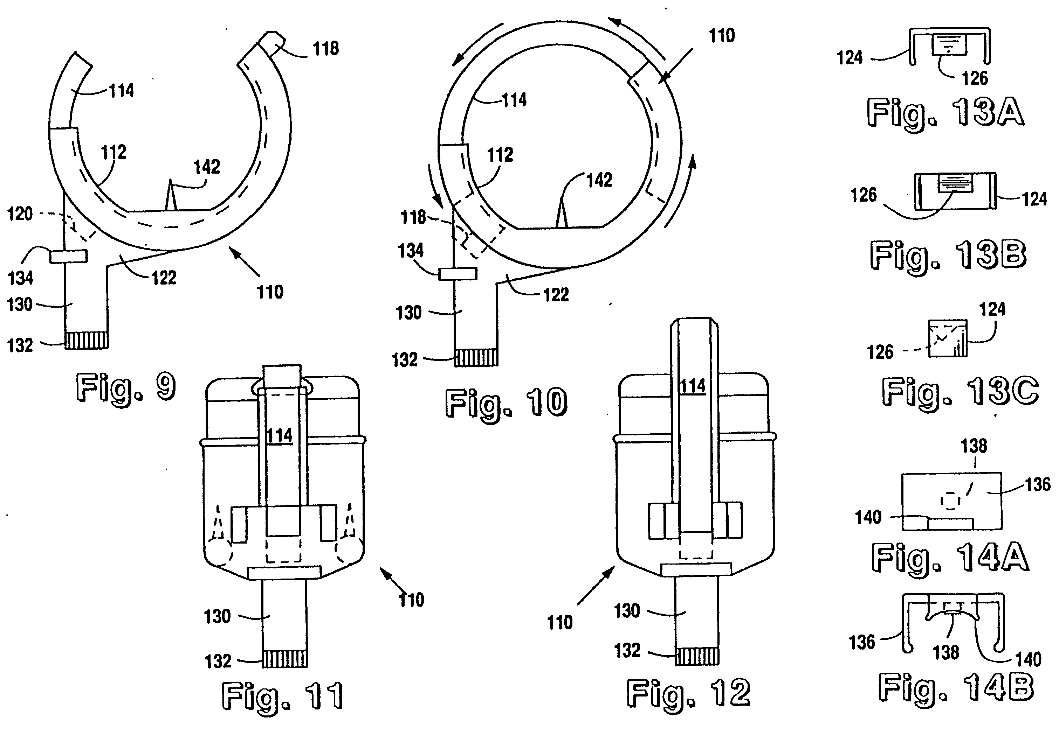

Referring to FIGS. 9, 10, 11 and 12, an alternative (and preferred) upper fixation device (the auto-lock rib carriage) is identified generally by the reference numeral (110). Auto-lock rib carriage (110) includes an approximately 3/4 circle under carriage ring (112) with a slidably journaled over carriage ring (114) movably engaged therewith. As is clear comparing FIGS. 9 and 10, simply rotating the over carriage ring (114) relative to the under carriage ring (112) forms (or alternatively opens) a fully enclosed circle.

Use of the auto-lock rib carriage (110) greatly simplifies attachment of a thoracodorsal distractor as compared with use of rods (20) as described above with reference to distractor (10). One simply positions the auto-lock rib carriage (110) in proper position and orientation relative to the designated upper attachment rib (12), moves the over carriage ring (114) so as to close the circle about the rib (12), and locks the over carriage ring (114) in place.

Referring principally to FIGS. 9, 10, 13A, 13B and 13C, over carriage ring (114) has a lip (118) which is sized for reception into a slot (120) formed in the base (122) of the auto-lock rib carriage (110). A saddle lock (124) with a locking cam (126) is designed to lock into position on the base (122) at an interlock site whereby the locking cam (126) securely holds the over carriage ring (114) in its closed position.

Referring principally to FIGS. 9, 10, 11 and 12, the under carriage (112) includes a carriage shaft (130) a terminal portion of which is formed into a spline (132). In an unshown embodiment of a thoracodorsal distractor which employs the auto-lock rib carriage (110), the upper end of the distractor sleeve (18) is formed into a socket which is suitably contoured to effectively mate with the spline portion (132) of the carriage shaft (130). This arrangement, among other benefits, allows the user to orient the auto-lock rib carriage (110) in any of numerous positions relative to the distractor sleeve (18). This is a particularly important benefit, in light of the potentially unpredictable course and orientation of ribs (12) in a scoliosis patient to which a user may want to attach the distractor at the upper end.

Referring principally to FIGS. 9, 10, 14A and 14B, the carriage shaft (130) has a shaft lip (134) which, in cooperation with a carriage shaft lock (136), serves to securely lock the auto-lock rib carriage (110) and the suitably modified distractor sleeve (not separately shown in the drawings) together. The carriage shaft lock (136) having, as it does, a shaft lock pin (138), is designed to snap into position relative to a recipient hole, analogous to hole (28) as described above (not shown in the drawings) on the distractor sleeve (18) in the same manner as the distraction locks (32) as described above. The carriage shaft lock (136) also exhibits a shaft lip clamp (140) which is sized and shaped to juxtapose the carriage shaft (130) on the side of the shaft lip (134) opposite the spline (118). The recipient hole in distractor sleeve (18) is positioned relative to the upper terminus of the distractor sleeve (18), so that the shaft lip clamp (140) is positioned relative to the shaft lock pin (138) such that, when the carriage shaft (130) is fully received within the socket of the distractor sleeve (18), and the carriage shaft lock (136) is pressed into position, the interaction of the shaft lip clamp (140) and the shaft lip (134) prevents separation of the auto-lock rib carriage (110) from the distractor sleeve (18).

Referring principally to FIGS. 9 and 10, to prevent lateral shifting of the auto-lock rib carriage (110) relative to the natural rib (12) to which is attached, a rib spike (142) is positioned on the base (122) of the auto-lock rib carriage (110) projecting into the circle defined by the over carriage ring (114) and the under carriage ring (112). While this will retard some movement of a thoracodorsal distractor relative to a natural rib (12) to which it is attached, and will, therefore, reduce some of the potential energy dissipation as discussed above, the ability of the natural rib (12) to oscillate within the confines of the circle defined by the over carriage ring (114) and the under carriage ring (112) in response to everyday forces will dissipate energy and promote work hypertrophy to a very beneficial degree as compared with any imaginable rigidly fixed systems.

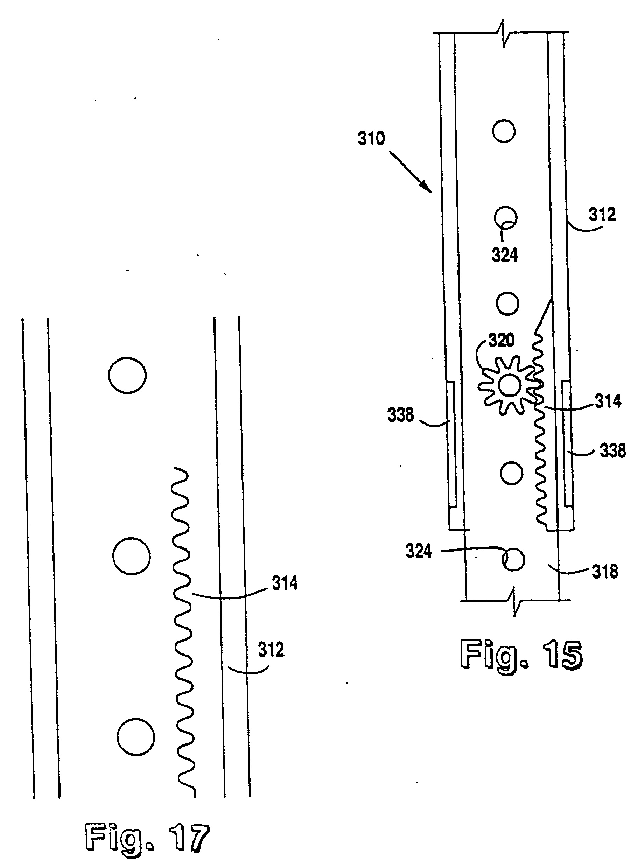

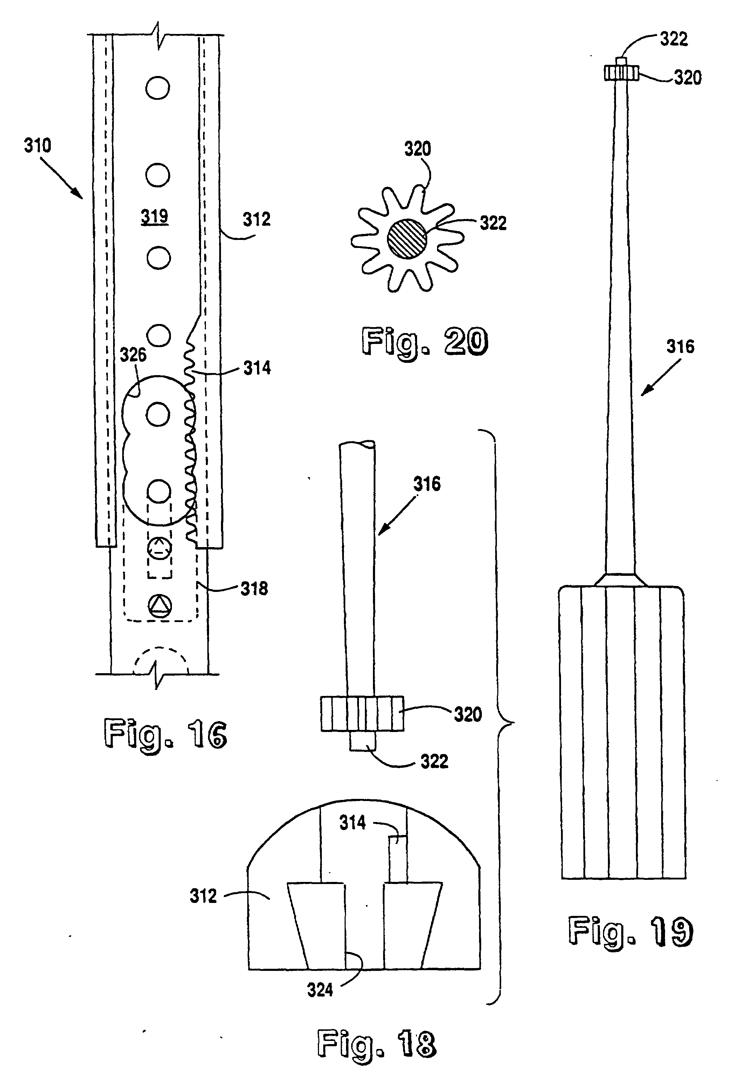

Referring principally to FIGS. 15, 16, 17, 18, 19 and 21, relevant portions of a preferred embodiment of a thoracodorsal distractor (310) as relates to the means for expanding and contracting the over-all length of the device is shown. The principle object served by this aspect of the system is to allow for post-implantation length adjustment by way of very simple, out-patient surgery.

The expansion/retraction system provided in FIGS. 15, 16, 17, 18, 19 and 21 is based, in part, upon a rack and pinion―like system. A portion of the internal margin of the distractor sleeve (312) is formed into a gear track or rack (314). The rack (314) is positioned superior to the channel through which the distractor shaft (318) extends and inferior to the top face (319) of the distractor sleeve (312) (partially removed in FIG. 16) for visibility.

A screwdriver-like probe (316) (see FIG. 19) exhibits a gear (320) at its lower end which gear (320) is sized and configured to mate with the teeth of rack (314). Extending axially from exposed face of the gear (320) is a pin (322) which is sized and shaped for insertion into one of the distraction holes (324) in the distractor shaft (318).

The top face (319) of the distractor sleeve (312) exhibits a three lobed orifice (326), each lobe of which is sized for receiving gear (320) therethrough for engagement with the rack (314). The margins of each lobe of orifice (326) is also threaded for threadingly mating with a locking system (to be described later). The lobes of the orifice (326) are configured whereby the gear (320) may be engaged or disengaged from the rack (314) of the distractor sleeve (312) and from the distractor shaft (318) at 0.5 cm intervals.

Operation of the expansion/retraction system of FIGS. 15, 16, 17, 18, 19 and 21 merely involves inserting the gear (320) of the probe (316) through the medial-most lobe of orifice (326), axially rotating the probe (316) in a counter-clockwise direction (from the perspective of the user), and removing the probe (316) from either of the two adjacent lobes of orifice (326) (depending on the extent of expansion desired). Of course, if more than 1.5 cm of expansion is desired, the process can be repeated.

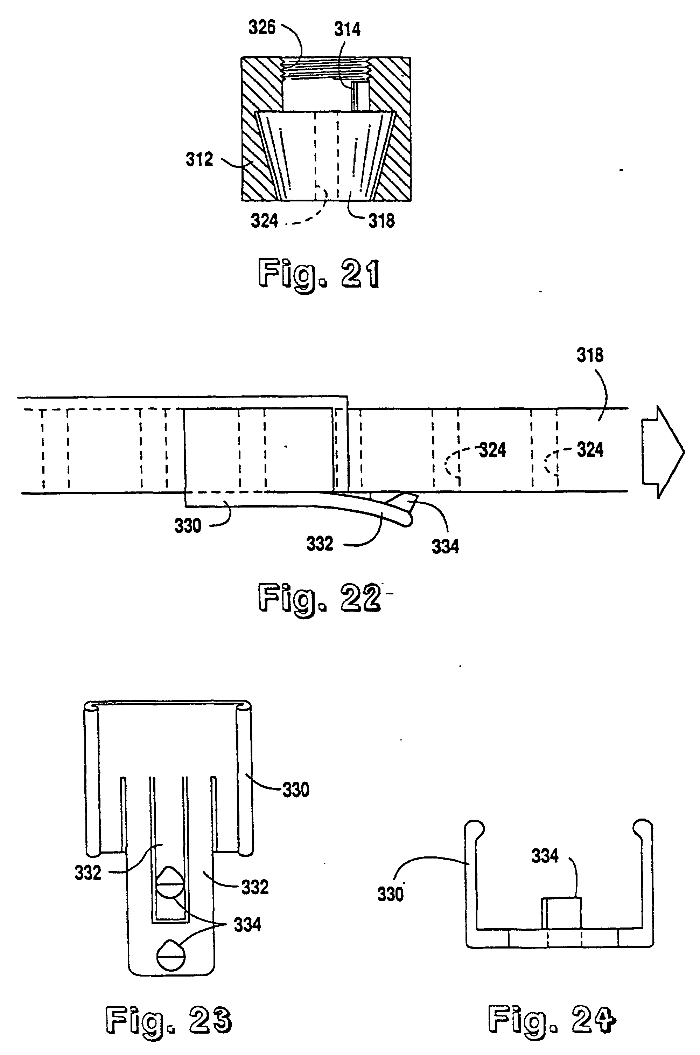

Referring to FIGS. 22, 23 and 24, Applicant provides a ratchet mechanism (330) for affixation to the distractor sleeve (312). The ratchet mechanism (330) includes dual leaves (332) each of which exhibits a locking cam (334) sized and shaped for extending into one of the distraction holes (324) in the distractor shaft (318) (as allowed through a suitably sized and shaped portal in the top face (319) of the distractor sleeve (312).

Referring principally to FIG. 22, the locking cams (334) are each contoured such that, when the ratchet mechanism (330) is oriented as shown in FIG. 22, they allow expansion, but not contraction of the distractor (310). In other words, each locking cam (334) exhibits a flattened face which is oriented toward the upper end of the distractor (310), but an obliquely oriented face which is oriented toward the upper end of the distractor (310). The operation and effect of the ratchet mechanism (330) is self evident from the figures.

Referring principally to FIG. 15, as with the distraction locks (32) as described above, the ratchet mechanism (330) is configured to snap onto the under surface of the prosthesis into indentations (338) formed on the outer surface of the distractor sleeve (312). This design permits a user during initial implantation to freely move the distractor (310) and only after reaching the appropriate minimal length thereof, affixing the ratchet mechanism (330) to prevent inadvertent contraction.

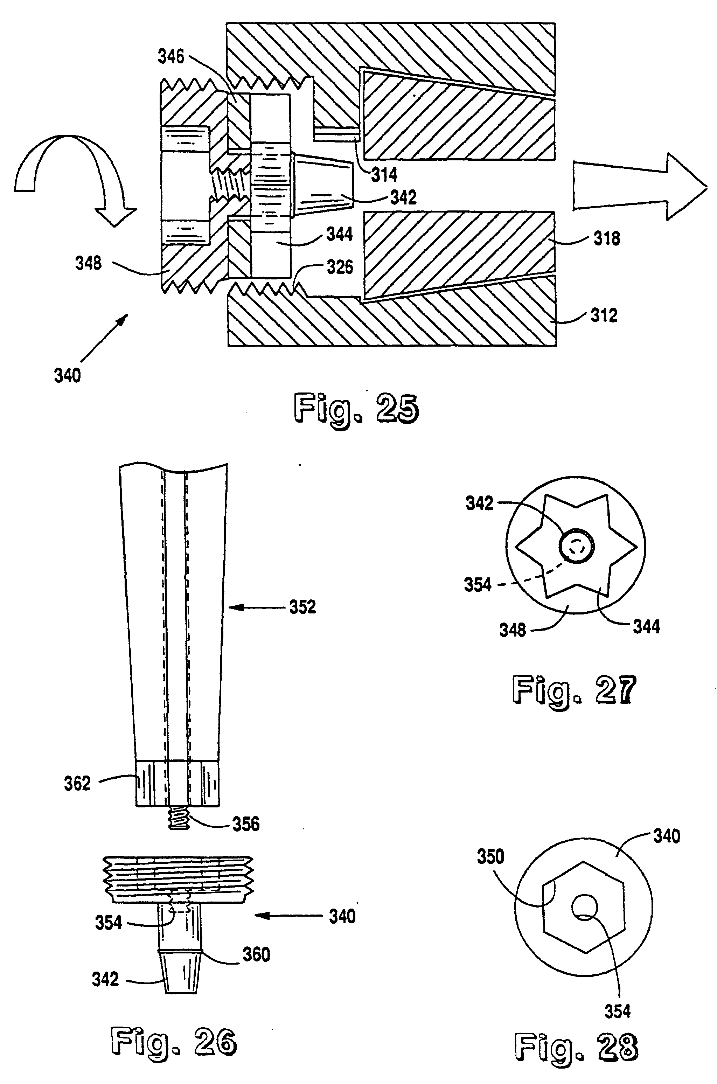

Referring principally to FIGS. 25, 26, 27 and 28, Applicant's design further includes a locking system for use with the just-described expansion/retraction system. This system centers on a locking disk (340) which includes a locking disk pin (342), a free gear (344), a lock washer (346), and a threaded cap disk (348). The threaded cap disk (348) has a hexagonal recess (350) in its exterior face for mating with a complimentarily shaped and sized tip (362) of a hex screwdriver (352). From the base of the hexagonal recess (350) is a threaded column (354) which is sized for threadingly mating with a threaded rod (356) which extends axially through the center of the hex screwdriver (352). The locking disk pin (342) is, in the preferred embodiment, an integral part of the threaded cap disk (348) and includes an annular gear-retaining bead (360) for maintaining the free gear (344) thereon with the lock washer (346) intervening the free gear (344) and the interior face of the threaded cap disk (348). The locking disk pin (342) is sized and shaped for extending into one of the distraction holes (324) in the distractor shaft (318) and is tapered so as to relieve tension from the ratchet mechanism (330) between adjustments of the distractor (310).

Once the distractor (310) is adjusted for length as desired as described above, a user of Applicant's system secures the threaded cap disk (348) to the hex screwdriver (352) by use of the threaded rod (356). The user then directs the locking disk (348) toward the medial most lobe of the orifice (326) on the distractor sleeve (312) and appropriately rotates the hex screwdriver (352) until the threaded cap disk (348) is fully seated in the orifice (326) whereby the locking disk pin (342) extends into the underlying hole (324) in the distractor shaft (318), the free gear (344) is mated with the rack (314) of the distractor sleeve (312) and lock washer (346) is compressed to the point were the threaded disk cap (348) will not accidentally become disengaged from the distractor sleeve (312). The user then disengages the hex screwdriver. (352) from the threaded cap disk (348) and closes the puncture wound used to access the distractor (310).

## Claims

1. Apparatus for attachment between human ribs comprising a first element (14, 18, 312) having a first attachment means (20, 110) for attachment to a first human rib (12) and a second element (16, 218, 318) which is telescopically received within a portion (18, 312) of the first element (14, 18, 312) and has a second attachment means (20, 110, 214) for attachment to a second human rib (12) **characterised in that** the apparatus is a thoracodorsal distractor (10, 210, 310) in which the first attachment means (20, 110) is for attachment to a upper thoracic human rib (12) immediately adjacent to the spine and the second attachment means (20, 110, 214) is for attachment to a lower human rib (12) adjacent to the spine, a first upper length (14) of the distractor (10) is contoured to define a first radius of curvature, and a second lower length (16a) of the distractor (10) is contoured to define a second radius of curvature which is larger than the first radius of curvature.

2. Apparatus, as in Claim 1, wherein each attachment means (20) comprises at least one rod (20) affixed at the outer end of the respective element (14, 18, 312 or 16, 218, 318), each rod (20) being made of malleable material and being of a length whereby it may be manipulated to loosely encircle a human rib (12).

3. Apparatus, as in Claim 1, wherein each attachment means (20) comprises a pair of rods (20) affixed at the outer end of the respective element (14, 18, 312 or 16, 218, 318), each pair of rods being made of a malleable material and being of a length whereby they may be manipulated to loosely encircle a human rib (12).

4. Apparatus, as in Claim 1, wherein the first attachment means (110) comprises first and second members (112, 114) which are relatively movable between a first position in which they define a closed circle of a size for loosely encircling the human rib and a second position in which they define a broken circle having a breach of sufficient size to admit the upper human rib (12).

5. Apparatus, as in any preceding claim, further comprising locking means (32, 334, 342) for selectively arresting telescopic movement between the first element (14, 18, 312) and the second element (16, 218, 318) at each of a plurality of post-operatively selectable positions (26, 324).

6. Apparatus, as in any preceding claim, further comprising at least one rib sling (217) for attaching a medial portion of the apparatus to a third human rib underlying the medial portion and for forcibly drawing a portion of the third rib to a position adjacent to the apparatus.

7. Apparatus, as in any preceding claim, in which a rack and pinion system (314, 320, 324) is operatively arranged to react between the first element (312) and the second element (318) to adjust the spacing between the first attachment means (20, 110) and the second attachment means (20, 110, 214).

8. Apparatus, as in Claim 7, in which the pinion (320) is removable after the spacing has been adjusted.

9. Apparatus, as in Claim 7 or 8 including a locking means (334, 342) arranged to lock the first element (312) and the second element (318) together after the spacing between the first adjustment means (20, 110) and the second attachment means (20, 110, 214) has been adjusted.

10. Apparatus, as in Claim 9, in which the locking means (334) is a ratchet mechanism (334) which inhibits contraction of the distractor (10, 210, 310) whilst allowing its extension.

11. Apparatus, as in Claim 9, in which the locking means (342) is a locking disc (340) arranged to lock the first element (312) to the second element (318) at each of a plurality of post-operatively selectable positions.

## Patentansprüche

1. Vorrichtung zur Befestigung zwischen menschlichen Rippen, die ein erstes Element (14, 18, 312) mit einer ersten Befestigungseinrichtung (20, 110) zur Befestigung an einer ersten menschlichen Rippe (12) und ein zweites Element (16, 218, 318) umfaßt, das teleskopartig in einem Teil (18, 312) des ersten Elementes (14, 18, 312) aufgenommen ist und eine zweite Befestigungseinrichtung (20, 110, 214) zur Befestigung an einer zweiten menschlichen Rippe (12) umfaßt, **dadurch gekennzeichnet, daß** die Vorrichtung eine thorakodorsale Streckvorrichtung (10, 210, 310) ist, bei der die erste Befestigungseinrichtung (20, 110) zur Befestigung an einer oberen menschlichen Rippe (12) des Brustkorbs in unmittelbarer Nachbarschaft des Rückgrats und die zweite Befestigungseinrichtung (20, 110, 214) für eine Befestigung an einer unteren menschlichen Rippe (12) in unmittelbarer Nähe des Rückgrats dient, wobei ein erstes oberes Stück (14) der Streckvorrichtung (10) so konturiert ist, daß es einen ersten Krümmungsradius definiert, und ein zweites unteres Stück (16a) der Streckvorrichtung (10) so konturiert ist, daß es einen zweiten Krümmungsradius definiert, der größer ist als der erste Krümmungsradius.

2. Vorrichtung nach Anspruch 1, bei der jede Befestigungseinrichtung (20) mindestens einen Stab (20) umfaßt, der am äußeren Ende des jeweiligen Elementes (14, 18, 312 oder 16, 218, 318) befestigt ist, wobei jeder Stab (20) aus einem verformbaren Material hergestellt ist und eine Länge besitzt, durch die er so manipuliert werden kann, daß er eine menschliche Rippe (12) lose umschließt.

3. Vorrichtung nach Anspruch 1, bei der jede Befestigungseinrichtung (20) zwei Stäbe (20) umfaßt, die am äußeren Ende des jeweiligen Elementes (14, 18, 312 oder 16, 218, 318) befestigt sind, wobei jedes Stäbepaar aus einem verformbaren Material hergestellt ist und eine solche Länge besitzt, daß sie so manipuliert werden können, daß sie eine menschliche Rippe (12) lose umschließen.

4. Vorrichtung nach Anspruch 1, bei der die erste Befestigungseinrichtung (110) erste und zweite Elemente (112, 114) umfaßt, die zwischen einer ersten Position, in der sie einen geschlossenen Kreis mit einer Größe zum losen Umschließen der menschlichen Rippe definieren und einer zweiten Position relativ bewegbar sind, in der sie einen Teilkreis definieren, der eine Eintrittsöffnung ausreichender Größe besitzt, um das Hindurchtreten der oberen menschlichen Rippe (12) zu ermöglichen.

5. Vorrichtung nach einem der vorhergehenden Ansprüche, die weiterhin Verriegelungsmittel (32, 334, 342) zum wahlweisen Festlegen der Teleskopbewegung zwischen dem ersten Element (14, 18, 312) und dem zweiten Element (16, 218, 318) an einer beliebigen einer Vielzahl von postoperativ auswählbaren Positionen (26, 324) ermöglicht.

6. Vorrichtung nach einem der vorhergehenden Ansprüche, die weiterhin wenigstens eine Rippenschlinge (217) zur Befestigung eines mittleren Teils der Vorrichtung an einer dritten menschlichen Rippe umfaßt, die unter dem mittleren Teil liegt, und zum zwangsweisen Ziehen eines Teils der dritten Rippe in eine Position in der Nähe der Vorrichtung.

7. Vorrichtung nach einem der vorhergehenden Ansprüche, in der ein Zahnrad/Zahnstangen-System (314, 320, 324) in betriebsmäßiger Weise so angeordnet ist, daß es zwischen dem ersten Element (312) und dem zweiten Element (318) wirkt, um den Abstand zwischen den ersten Befestigungseinrichtungen (20, 110) und den zweiten Befestigungseinrichtungen (20, 110, 214) einzustellen.

8. Vorrichtung nach Anspruch 7, bei der das Zahnrad entfernbar ist, nachdem der Abstand eingestellt worden ist.

9. Vorrichtung nach Anspruch 7 oder 8, mit einer Verriegelungseinrichtung (334, 342), die so ausgebildet ist, daß sie das erste Element (312) und das zweite Element (318) miteinander verriegelt, nachdem der Abstand zwischen den ersten Befestigungseinrichtungen (20, 110) und den zweiten Befestigungseinrichtungen (20, 110, 214) eingestellt worden ist.

10. Vorrichtung nach Anspruch 9, bei der die Verriegelungseinrichtung (334) ein Rastmechanismus (334) ist, der eine Kontraktion der Streckvorrichtung (10, 210, 310) verhindert, seine Verlängerung jedoch ermöglicht.

11. Vorrichtung nach Anspruch 9, bei der die Verriegelungseinrichtung (342) eine Verriegelungsscheibe (340) ist, die so angeordnet ist, daß sie das erste Element (312) am zweiten Element (318) an einer beliebigen einer Vielzahl von postoperativ wählbaren Positionen verriegelt.

## Revendications

1. Appareil destiné à être fixé entre des côtes humaines, comprenant un premier élément (14, 18, 312) ayant un premier moyen de fixation (20, 110) en vue d'une fixation à une première côte humaine (12) et un second élément (16, 218, 318) qui est reçu de façon télescopique à l'intérieur d'une partie (18, 312) du premier élément (14, 18, 312) et possède un second moyen de fixation (20, 110, 214) en vue d'une fixation à une seconde côte humaine (12), **caractérisé par le fait que** l'appareil est un distracteur thoraco-dorsal (10, 210, 310) dans lequel le premier moyen de fixation (20, 110) est adapté à la fixation à une côte humaine thoracique supérieure (12) immédiatement adjacente à la colonne vertébrale et le second moyen de fixation (20, 110, 214) est adapté à la fixation à une côte humaine inférieure (12) adjacente à la colonne vertébrale, une première longueur supérieure (14) du distracteur (10) est configurée pour définir un premier rayon de courbure, et une seconde longueur inférieure (16a) du distracteur (10) est configurée pour définir un second rayon de courbure qui est plus grand que le premier rayon de courbure.

2. Appareil selon la revendication 1, dans lequel chaque moyen de fixation (20) comprend au moins une tige (20) fixée à l'extrémité externe de l'élément respectif (14, 18, 312 ou 16, 218, 318), chaque tige (20) étant faite d'un matériau malléable et étant d'une longueur par laquelle elle peut être manipulée pour entourer sans serrer une côte humaine (12).

3. Appareil selon la revendication 1, dans lequel chaque moyen de fixation (20) comprend une paire de tiges (20) fixées à l'extrémité externe de l'élément respectif (14, 18, 312 ou 16, 218, 318), chaque paire de tiges étant faite d'un matériau malléable et étant d'une longueur par laquelle elles peuvent être manipulées pour entourer sans serrer une côte humaine (12).

4. Appareil selon la revendication 1, dans lequel le premier moyen de fixation (110) comprend des premier et second éléments (112, 114) qui sont relativement mobiles entre une première position dans laquelle ils définissent un cercle fermé d'une dimension permettant d'entourer sans serrer la côte humaine et une seconde position dans laquelle ils définissent un cercle interrompu ayant une ouverture de dimension suffisante pour recevoir la côte humaine supérieure (12).

5. Appareil selon l'une quelconque des revendications précédentes, comprenant en outre un moyen de blocage (32, 334, 342) pour arrêter de façon sélective le mouvement télescopique entre le premier élément (14, 18, 312) et le second élément (16, 218, 318) au niveau de. chacune d'une pluralité de positions sélectionnables après l'opération (26, 324).

6. Appareil selon l'une quelconque des revendications précédentes, comprenant en outre au moins une écharpe costale (217) pour fixer une partie médiane de l'appareil à une troisième côte humaine se situant au-dessous de la partie moyenne et pour tirer de force une partie de la troisième côte jusqu'à une position voisine de l'appareil.

7. Appareil selon l'une quelconque des revendications précédentes, dans lequel un système à crémaillère et pignon (314, 320, 324) est disposé à l'opération pour réagir entre le premier élément (312) et le second élément (318) afin d'ajuster l'espacement entre le premier de fixation (20, 110) et le second moyen de fixation (20, 110, 214).

8. Appareil selon la revendication 7, dans lequel le pignon (320) est susceptible d'être retiré après que l'espacement ait été ajusté.

9. Appareil selon l'une des revendications 7 ou 8, comprenant un moyen de blocage (334, 342) disposé pour bloquer le premier élément (312) et le second élément (318) ensemble après que l'espacement entre le premier moyen de fixation (20, 110) et le second moyen de fixation (20, 110, 214) ait été ajusté.

10. Appareil selon la revendication 9, dans lequel le moyen de fixation (334) est un mécanisme à cliquet (334) qui empêche la contraction du distracteur (10, 210, 310) tout en permettant son extension.

11. Appareil selon la revendication 9, dans lequel le moyen de blocage (342) est un disque de blocage (340) disposé pour bloquer le premier élément (312) au second élément (318) au niveau de chacune d'une pluralité de positions sélectionnables après l'opération.
